# EUROPÄISCHE PATENTANMELDUNG

(11) **EP 1 422 212 A1**
(43) Veröffentlichungstag der Anmeldung: **26.05.2004**
(21) Anmeldenummer: 03025073.2
(22) Anmeldetag: 03.11.2003
(51) Int. Cl.: C07C 45/54, C07C 49/597

(54) **Verfahren zur Herstellung von Cyclopentenonen**

(30) Priorität: 25.11.2002 DE 10254853
(71) Anmelder: BASF AKTIENGESELLSCHAFT, 67056 Ludwigshafen (DE)
(72) Erfinder: Liang, Shelue, Dr., 67071 Ludwigshafen (DE); Fischer, Rolf-Hartmuth, Dr., 69121 Heidelberg (DE); Huber-Dirr, Sylvia, Dr., 64673 Zwingenberg (DE); Haunert, Andrea, Dr., 68163 Mannheim (DE)

(57) **Zusammenfassung**

Gegenstand der vorliegenden Erfindung ist ein Verfahren zur Herstellung von 2-Cyclopentenonen der allgemeinen Formel: 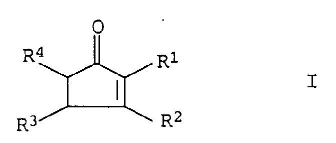 in der R¹ bis R⁴ Wasserstoffatome bedeuten oder für Alkyl- oder Alkenylreste mit 1 bis 12 Kohlenstoffatomen, Cycloalkyl- oder Cycloalkenylreste mit 5 bis 7 Kohlenstoffatomen, Aralkylen- oder Arylreste stehen, durch Umsetzung von Hexendisäuren und/oder deren Ester der allgemeinen Formeln 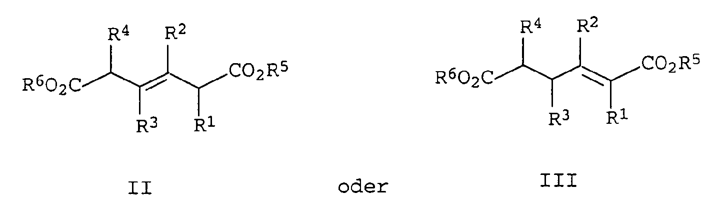 in denen R¹ bis R⁴ die obengenannte Bedeutung haben und R⁵ und R⁶ Wasserstoffatome bedeuten oder für Alkylreste mit 1 bis 12 Kohlenstoffatomen, Cycloalkylreste mit 5 oder 6 Kohlenstoffatomen, Aralkyl- oder Arylreste stehen, bei Temperaturen von 150 bis 450°C an festen, oxidischen Katalysatoren, dadurch gekennzeichnet, dass die Katalysatoren auf einem oxidischen Trägermaterial 0,01 bis 5 Gew.-% eines Alkalioxids aufweisen.

## Beschreibung

Die vorliegende Erfindung betrifft ein Verfahren zur Herstellung von 2-Cyclopentenonen durch Umsetzung von 2- oder 3-Hexen-1,6-dicarbonsäuren oder deren Estern in Gegenwart von heterogenen Katalysatoren, die aus Alkalioxiden auf Katalysator-Trägern bestehen.

Die Synthese von 2-Cyclopentenonen durch Umsetzung von substituierten oder unsubstituierten 2- oder 3-Hexen-1,6-dicarbonsäuren oder ihren Estern an festen oxidischen Katalysatoren bei 150°C bis 450°C ist aus EP-A 297 447 bekannt. Verwendet werden feste oxidische Katalysatoren der I bis V Hauptgruppe, der I bis VIII Nebengruppe des periodischen Systems der Elemente oder Oxide der seltenen Erdmetalle oder Gemische der genannten Oxide. Besonders bevorzugt wird die Umsetzung in der Gasphase unter Verwendung ° eines Katalysator-Wirbelbetts durchgeführt. Gemäß den vier Ausführungsbeispielen der EP-A 297 447 wird als Katalysator besonders bevorzugt γ-Aluminiumoxid bzw. mit Bariumoxid dotiertes Aluminiumoxid verwendet.

Die Versuche in den vier Ausführungsbeispielen wurden bei 345°C/Normaldruck in Gegenwart von Wasserdampf, Stickstoff als Trägergas und γ-Aluminiumoxid- oder γ-Aluminiumoxid/Bariumoxid-Wirbelbett-Katalysatoren jeweils 6 Stunden lange betrieben. Die höchste Ausbeute bei Verwendung von γ-Aluminiumoxid betrug 51 % (Selektivität 65 %, Beispiel 1), bei Verwendung von γ-Aluminiumoxid/10 % Bariumoxid 55 % (Selektivität 69 %, Beispiel 4).

Es bestand die Aufgabe, das Verfahren zur Herstellung von Cyclopentenonen aus 2- oder 3-Hexen-1,6-dicarbonsäuren oder deren Ester, insbesondere im Hinblick auf die Cyclopentenonselektivität durch das Auffinden noch besserer Katalysatoren weiter zu verbessern. Mit einer hohen Cyclopentenonselektivität sollte eine möglichst hohe Cyclopentenon-Ausbeute verbunden sein, um im Verfahren möglichst wenig 2- oder 3-Hexen-1,6-dicarbonsäuren oder deren Ester zurückführen zu müssen. Weiterhin sollten die Katalysatoren eine hohe Standzeit aufweisen.

Diese Aufgabe wird gelöst in einem Verfahren zur Herstellung von 2-Cyclopentenonen der allgemeinen Formel 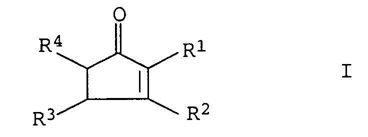 in der R¹ bis R⁴ Wasserstoffatome bedeuten oder für Alkyl- oder Alkenylreste mit 1 bis 12 Kohlenstoffatomen, Cycloalkyl- oder Cycloalkenylreste mit 5 bis 7 Kohlenstoffatomen, Aralkylen- oder Arylreste stehen, durch Umsetzung von Hexendisäuren und/oder deren Ester der allgemeinen Formeln 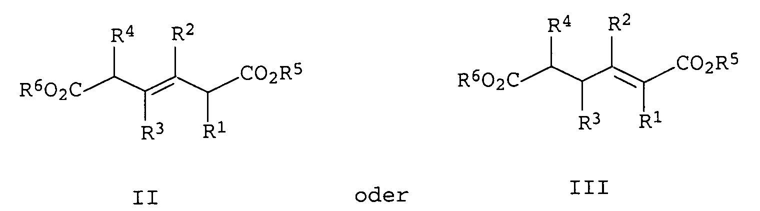 in denen R¹ bis R⁴ die obengenannte Bedeutung haben und R⁵ und R⁶ Wasserstoffatome bedeuten oder für Alkylreste mit 1 bis 12 Kohlenstoffatomen, Cycloalkylreste mit 5 oder 6 Kohlenstoffatomen, Aralkyl- oder Arylreste stehen, bei Temperaturen von 150 bis 450°C an festen, oxidischen Katalysatoren, welche auf einem oxidischen Trägermaterial 0,01 bis 5 Gew.-%, bevorzugt 0,1 bis 3 Gew.-%, besonders bevorzugt 0,3 bis 2 Gew.-%, mindestens eines Alkalioxids aufweisen. Gewichtsprozentangaben beziehen sich jeweils auf den gesamten Katalysator aus Aktivmetalloxid und Trägermaterial.

Als Alkalioxide kommen Lithium, Natrium-, Kalium-, Rubidium-, und Caesiumoxid oder deren Gemische in Frage. Besonders bevorzugt sind Natrium- und Kaliumoxid als katalytisch aktive Masse.

Als Trägermaterial werden Metalloxide der II bis V Hauptgruppe, der I bis VIII Nebengruppe des periodischen Systems der Elemente oder Oxide der seltenen Erdmetalle oder Gemische derselben verwendet. Beispiele für derartige Träger sind Magnesiumoxid, Calciumoxid, Bariumoxid, weiterhin Bortrioxid, Aluminiumoxid, Siliciumoxid, z.B. in Form von Kieselgel, Kieselgur oder Quarz, ferner Zinndioxid, Bismutoxid, Kupferoxid, Zinkoxid, Lanthanoxid, Titandioxid, Zirkondioxid, Vanadiumoxide, Chromoxide, Molybdänoxide, Wolframoxide, Manganoxide, Eisenoxide, Ceroxide, Neodymoxide oder Gemische derartiger Oxide.

Bevorzugt werden als Trägermaterial Aluminiumoxid und/oder Siliziumoxid verwendet.

Die erfindungsgemäß verwendeten Trägerkatalysatoren können nach an sich bekannten Verfahren, beispielsweise durch Fällung der katalytisch aktiven Komponente aus ihren Salzlösungen in Gegenwart des Trägermaterials durch Zugabe von Alkalimetall-Hydroxid oder Carbonat-Lösungen hergestellt werden. Die jeweiligen Hydroxide, Oxidhydrate, basichen Salze oder Carbonate werden so ausgefällt.

Die Niederschläge werden anschließend getrocknet und durch Kalzinierung bei im allgemeinen 300 bis 1300°C; bevorzugt 400 bis 1200°C in die entsprechenden Oxide, Mischoxide und/oder gemischtvalenten Oxide umgewandelt.

Neben den oben genannten Fällungskatalysatoren, die als Trägerkatalysatoren eingesetzt werden können, sind auch Trägerkatalysatoren geeignet, bei denen die katalytisch aktiven Komponenten auf andere Art auf das Trägermaterial aufgebracht worden sind.

Beispielsweise können die katalytisch aktiven Komponenten durch _ Tränkung mit Lösungen oder Suspensionen der Salze oder Oxide der entsprechenden Elemente und Trocknung aufgebracht werden.

Die Trägerkatalysatoren können auch durch Vermischen des Trägers mit einem Alkalisalz und Wasser, Verkneten und Verstrangen des Gemischs und anschließendendes Trocknen und Calcinieren hergestellt werden.

Die katalytisch aktiven Komponenten können auch auf den Träger aufgebracht werden, indem der Träger mit Lösungen thermisch leicht zersetzbarer Salze imprägniert wird und der so behandelte Träger auf Temperaturen von 300 bis 600°C erhitzt wird, wobei die adsorbierten Metallverbindungen thermisch zersetzt werden.

Thermisch leicht zersetzbare Salze sind beispielsweise Nitrate und thermisch leicht zersetzbare Komplexverbindungen wie Carbonyl- oder Hydride-Komplexe der katalytisch aktiven Metalle. Die thermische Zersetzung wird vorzugsweise in einer Schutzgasatmosphäre durchgeführt. Geeignete Schutzgase sind z.B. Stickstoff, Kohlendioxid, Wasserstoff oder Edelgase.

Weiterhin kann die katalytisch aktive Komponente durch Aufdampfen oder Flammspritzen auf dem Trägermaterial abgeschieden werden.

Die erfindungsgemäße Reaktion läßt sich z.B. für die Umsetzung von 3-Hexen-1,6-dicarbonsäuredimethylester zu 2-Cyclopentenon durch die folgende Reaktionsgleichung darstellen:

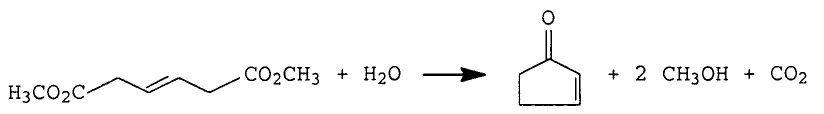

Als Ausgangsstoffe der Formeln II und III kommen 3-Hexen-1,6-dicarbonsäure oder 2-Hexen-1,6-disäure, die gegebenenfalls durch die Reste R¹ bis R⁴ substituiert sind in Betracht. Die Reste R¹ bis R⁴ können Alkyl- oder Alkenylreste mit 1 bis 12 C-Atomen, wie Methyl-, Ethyl-, Propyl-, Isopropyl-, Butyl-, Isobutyl-, tert.-Butyl-, Hexyl-, Nonyl-, Allyl-, Hexenyl- oder Nonenylreste, Cycloalkyl- oder Cycloalkenylreste mit 5 bis 7 C-Atomen, wie Cyclohexyl-, Cyclopentyl-, 2-Cyclohexenyl-, oder 2-Cyclopentenylreste, Aralkyl- oder Arylreste, wie Phenyl- oder Benzylreste sein. Die Ester der Formeln II und III sind aliphatische, cycloaliphatische, araliphatische oder aromatische Mono- oder Diester der genannten Dicarbonsäuren. Als Reste R⁵ und R⁶ seien z.B. Methyl-, Ethyl-, Propyl-, Isopropyl-, tert.-Butyl-, Hexyl-, Nonyl-, Dodecyl-, Cyclopentyl-, Cyclohexyl-, Phenyl- oder Benzylreste genannt.

Beispielsweise können folgende Ausgangsstoffe verwendet werden: 3-Hexen-1,6-disäure, 2-Hexen-1,6-disäure, 2-Methyl-3-hexen-1,6-disäure, 2,5-Dimethyl-3-hexen-1,6-disäure, 3,4-Dimethyl-3-hexen-1,6-disäure, 2-Allyl-3-hexen-1,6-disäure, 3-Cyclohexyl-2-hexen-1,6-disäure, 3-(2-Cyclopentyl)-3-hexen-1,6-disäure, 3-Phenyl-3-hexen-1,6-disäure und 2-Benzyl-3-hexen-1,6-disäure, 3-Hexen-1,6-disäuredimethylester, 2-Hexen-1,6-disäuredimethylester, 3-Hexen-1,6-disäuremonomethylester, 3-Hexen-1,6-disäurediethylester, 2-Hexen-1,6-disäuredibutylester, 3-Hexen-1,6-disäuredicyclohexylester, 3-Hexen-1,6-disäuredibenzylester, 2-Methyl-3-hexen-1,6-disäuredimethylester, 2,5-Dimethyl-3-hexen-1,6-disäuredimethylester, 3,4-Dimethyl-3-hexen-1,6-disäuredimethylester, 2-Allyl-3-hexen-1,6-disäuredimethylester, 3-Cyclohexyl-2-hexen-1,6-disäurediethylester, 3-(2-Cyclopentenyl)-3-hexen-1,6-disäuredimethylester, 3-Phenyl-3-hexen-1,6-disäurediethylester oder 2-Benzyl-3-hexen-1,6-disäuredimethylester. Die Umsetzung der Ester ist von besonderem technischen Interesse.

Es ist zwar möglich, die erfindungsgemäße Umsetzung ohne Zusatz von Wasser durchzuführen, allerdings wird durch die Zugabe von Wasser eine bemerkenswerte Erhöhung von Selektivität und Standzeit erreicht. Das Molverhältnis von Ausgangsstoff II oder III zu Wasser beträgt hierbei vorteilhaft 1:0,01 bis 1:20, insbesondere 1:0,5 bis 1:10.

Die Umsetzung kann in der Gasphase oder in flüssiger Phase, gegebenenfalls auch unter Mitverwendung von Verdünnungsmitteln durchgeführt werden. Besonders bevorzugt ist die Umsetzung als Festbettreaktion mit Festbettkatalysatoren in der Gasphase durchzuführen.

Die Umsetzung findet bei 200°C bis 450°C, vorzugsweise 250 bis 430°C, insbesondere bei 300°C bis 420°C statt. Im allgemeinen wird die Reaktion unter Atmosphärendruck durchgeführt. Es ist jedoch auch möglich, schwach verminderten oder schwach erhöhten Druck, z.B. bis zu 20 bar anzuwenden. Die Katalysatorbelastung liegt im allgemeinen bei 0,01 bis 40, vorzugsweise 0,1 bis 20 g Ausgangsstoff der Formel II und/oder III je Gramm Katalysator und Stunde.

Die Umsetzung in der Flüssigphase wird beispielsweise so durchgeführt, dass man ein Gemisch aus der Ausgangsverbindung und gegebenenfalls Wasser in Gegenwart eines suspendierten Festbettkatalysators auf die gewünschte Reaktionstemperatur erhitzt. Nach Ablauf der notwendigen Reaktionszeit wird das Reaktionsgemisch abgekühlt und der Katalysator, z.B. durch Filtration, entfernt. Das Reaktionsgemisch wird anschließend zur Gewinnung des Ketons bzw. des unumgesetzten Ausgangsstoffes fraktionierend destilliert.

Eine bevorzugte Ausführungsform des erfindungsgemäßen Verfahrens in der Gasphase sieht z.B. so aus, dass man ein Gemisch aus dem Ausgangsstoff der Formel II und/oder III und Wasser zunächst verdampft und dann, gegebenenfalls zusammen mit einem Inertgas, wie Stickstoff, Kohlendioxid oder Argon, bei der gewünschten Reaktionstemperatur gasförmig über einen fest angeordneten Katalysator leitet. Bei Festbettanordnung ist die Rieselfahrweise, bei der Gas und Flüssigkeit von oben nach unten über das katalysatische Festbett geführt werden, bevorzugt. Der Reaktionsaustrag wird mittels geeigneter Kühlvorrichtung kondensiert und anschließend durch fraktionierende Destillation aufgearbeitet. Unumgesetzter Ausgangsstoff kann zurückgeführt werden.

Die für das erfindungsgemäße Verfahren benötigten Ausgangsstoffe sind ausgehend von 1,4-Dicyan-2- oder 1,4-Dicyan-3-butenen durch Hydrolyse oder Umsetzung mit Alkohlen und Salzsäure (Pinner-Reaktion), durch Metathese von Alkencarbonsäureestern (siehe z.B. J. of Molecular Catalysis 8 (1980), S. 107 bis 11) oder durch katalytische Dimerisierung von Acrylestern z.B. mit Pd-Katalysatoren wie in J. Org. Chem. 48 (1983), S. 5364 bis 5366 beschrieben oder Rh-Katalysatoren (siehe EP-A 475 386) nicht nur leicht in guten Ausbeuten, sondern auch mit einem in weiten Grenzen variierbaren Substitutionsmuster herstellbar. Hierbei ist die Dimerisierung von Acrylestern besonders bevorzugt.

Aus EP-A 269 042 ist bekannt, 2- und 3-Pentensäuremethylester bei 130 bis 135°C in Gegenwart von starken Basen wie Natriummethylat, Natriumamid oder Natriumhydrid zu Gemischen aus 2-Propenylidenund 2-(1-Propenyl)-3-ethyl-glutarsäuredimethylester, also dimeren Pentensäureestern umzusetzen. Eine ähnliche Dimerisierung war bei der Umsetzung von 2- und 3-Hexendicarbonsäurediestern bei deutlich höheren Temperaturen und Alkalioxiden, also ebenfalls starken Basen, zu erwarten gewesen. Daher war es überraschend, dass gemäß Beispiel 1 Cyclopentenon-Ausbeuten von 85 % bei Selektivitäten von 91 % erzielt werden.

Die nach dem erfindungsgemäßen Verfahren erhältlichen 2-Cyclopentenone sind wertvolle Zwischenprodukte. Das α,β-ungesättigte Ketonsystem in den 2-Cyclopentenonen ermöglicht eine Vielzahl von Additionsreaktionen vom Michael- oder Diels-Alder-Typ. 2-Cyclopentenone stellen somit wertvolle und vielseitig verwendbare Ausgangsverbindungen für die Synthese von fünfgliedrigen Ringen dar.

Die nachfolgenden Beispiele sollen das erfindungsgemäße Verfahren näher erläutern.

### Beispiele

### Beispiel 1

### Herstellung des Cyclisierungskatalysators A (1 % K₂O/Al₂O₃)

3kg Al₂O₃ wurden mit 0,05 kg Kaliumcaronbatund 2,8 kg Wasser verknetet. Die gut durchmischte Masse wurde zu 4 mm Strängen geformt und bei 120°C getrocknet. Die getrockneten Stränge wurden bei 1200°C calciniert.

### Beispiel 2

### Cyclisierung von 3-Hexen-1,6-disäuredimethylester zu 2-Cyclopentenon

In einem elektrisch beheizten Gasphasenreaktor wurden 80 ml Katalysator A mit 20 ml Quarzringen als Verdampferstrecke überschichtet. Die Apparatur wurde in Rieselfahrweise betrieben. Pro Stunde wurden 0,008 kg 3-Hexen-1,6-disäuredimethylester und 0,004 ml Wasser verdampft und mit 20 1 Stickstoff bei 400°C über den Katalysator A geleitet. Die Katalysatorbelastung betrug 0,1 kg 2-Hexen-1,6-disäuredimethylester/1 Katalysator x Stunde.

Der gasförmige Reaktionsaustrag wurde unter Kühlung kondensiert und gaschromatographisch analysiert. Die 2-Cyclopentenon-Ausbeute betrug 85 %, die Selektivität 91 %.

## Patentansprüche

1. Verfahren zur Herstellung von 2-Cyclopentenonen der allgemeinen Formel: 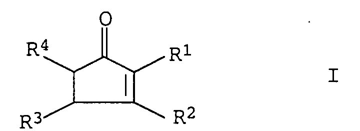 in der R¹ bis R⁴ Wasserstoffatome bedeuten oder für Alkyloder Alkenylreste mit 1 bis 12 Kohlenstoffatomen, Cycloalkyloder Cycloalkenylreste mit 5 bis 7 Kohlenstoffatomen, Aralkylen- oder Arylreste stehen, durch Umsetzung von Hexendisäuren und/oder deren Ester der allgemeinen Formeln 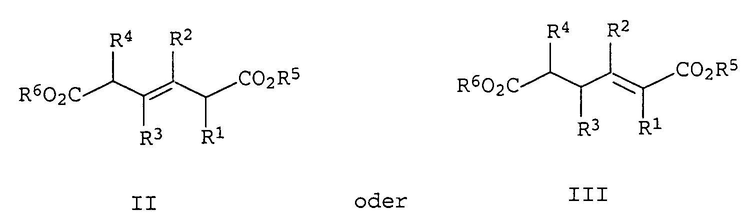 in denen R¹ bis R⁴ die obengenannte Bedeutung haben und R⁵ und R⁶ Wasserstoffatome bedeuten oder für Alkylreste mit 1 bis 12 Kohlenstoffatomen, Cycloalkylreste mit 5 oder 6 Kohlenstoffatomen, Aralkyl- oder Arylreste stehen, bei Temperaturen von 150 bis 450°C an festen, oxidischen Katalysatoren, **dadurch gekennzeichnet, dass** die Katalysatoren auf einem oxidischen Trägermaterial 0,01 bis 5 Gew.-% mindestens eines Alkalioxids aufweisen.

2. Verfahren nach Anspruch 1, **dadurch gekennzeichnet, dass** man als Trägermaterial Metalloxide der II bis V Hauptgruppe, der I bis VIII Nebengruppe des Periodensystems der Elemente, Oxide der seltenen Erdmetalle oder Gemische derselben verwendet.

3. Verfahren nach einem der Ansprüche 1 oder 2, **dadurch gekennzeichnet, dass** Aluminiumoxid und/oder Siliciumoxid als Trägermaterial verwendet werden.

4. Verfahren nach einem der Ansprüche 1 bis 3, **dadurch gekennzeichnet, dass** Natriumoxid und/oder Kaliumoxid verwendet werden.

5. Verfahren nach einem der Ansprüche 1 bis 4, **dadurch gekennzeichnet, dass** man die Umsetzung im Festbett durchführt.

6. Verfahren nach einem der Ansprüche 1 bis 5, **dadurch gekennzeichnet, dass** man die Ausgangsstoffe der allgemeinen Formeln II und III durch Dimerisierung von Acrylsäureestern herstellt.
